(19) Europäisches Patentamt
European Patent Office
Office européen des brevets

(11) Veröffentlichungsnummer: **0 256 420**
**A2**

(12) # EUROPÄISCHE PATENTANMELDUNG

(21) Anmeldenummer: **87111240.5**

(22) Anmeldetag: **04.08.87**

(51) Int. Cl.⁴: **C07D 213/38**, C07D 213/61, C07D 213/73, A23K 1/16

(30) Priorität: **14.08.86 DE 3627663**

(43) Veröffentlichungstag der Anmeldung: **24.02.88 Patentblatt 88/08**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Lindel, Hans, Dr.**
**Carl-Duisberg-Strasse 321**
**D-5090 Leverkusen 1(DE)**
Erfinder: **Hallenbach, Werner, Dr.**
**Kleiststrasse 10**
**D-4018 Langenfeld(DE)**
Erfinder: **Berschauer, Friedrich, Dr.**
**Claudiusweg 9**
**D-5600 Wuppertal 1(DE)**
Erfinder: **de Jong, Anno, Dr.**
**Stockmansmühle 46**
**D-5600 Wuppertal 1(DE)**
Erfinder: **Scheer, Martin, Dr.**
**Herberts-Katernberg 7**
**D-5600 Wuppertal 1(DE)**

(54) **Heteroarylethylamine, Verfahren zu ihrer Herstellung und ihre Verwendung als Leistungsförderer bei Tieren.**

(57) Die vorliegende Erfindung betrifft Heteroarylethylamine der Formel I

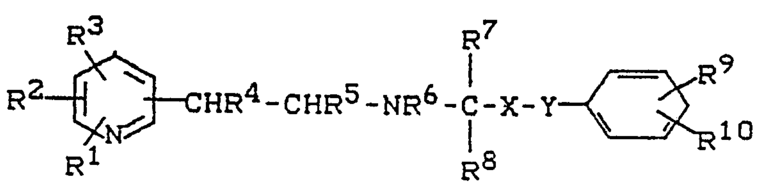

(I)

in welcher

$R^1$ für Wasserstoff, Alkyl, Halogen, Halogenalkyl, Hydroxy, Cyano, Alkoxycarbonyl, Aminocarbonyl, Mono-und Dialkylaminocarbonyl, Alkoxy, Halogenalkoxy, Halogenalkylthio, $NHSO_2$-Alkyl steht,

$R^2$ für Wasserstoff, Hydroxy, Alkoxy oder den Rest $-NR^{11}R^{12}$ steht,

$R^3$ für die bei $R^1$ angegebenen Reste steht,

$R^4$ für Hydroxy, Acyloxy oder Alkoxy steht,

EP 0 256 420 A2

$R^5$ für Wasserstoff oder Alkyl steht,

$R^6$ für Wasserstoff oder Alkyl steht,

$R^7$ für Wasserstoff oder Alkyl steht,

$R^8$ für Wasserstoff oder Alkyl steht,

X für $C_1$-$C_{10}$-Alkylen oder eine direkte Bindung steht,

Y für Sauerstoff oder eine direkte Bindung steht,

$R^9$ für Wasserstoff, $C_1$-$C_{10}$-Alkyl, welches gegebenenfalls durch Hydroxy, Alkoxy, Acyloxy oder den Rest $-NR^{13}R^{14}$ substituiert ist, sowie für den Rest $COR^{15}$ oder den Rest O-Z-$R^{16}$ steht,

Z für $C_1$-$C_{10}$-Alkylen, -Alkenylen oder -Alkinylen steht,

$R^{10}$ für Wasserstoff, Halogen, Alkyl, Alkoxy, Hydroxy oder den Rest $COR^{15}$ steht,

$R^{11}$ für Wasserstoff oder Alkyl steht,

$R^{12}$ für Wasserstoff, Alkyl, Halogenalkyl oder Acyl steht,

$R^{13}$ für Wasserstoff, gegebenenfalls substituiertes Alkyl, gegebenenfalls substituiertes Aryl steht,

$R^{14}$ für die bei $R^{13}$ angegebenen Reste steht,

$R^{15}$ für Hydroxy, Alkoxy oder den Rest $NR^{13}R^{14}$ steht,

$R^{16}$ für Hydroxy, Alkoxy, Acyloxy, den Rest $NR^{13}R^{14}$ oder den Rest $COR^{15}$ steht,

sowie ihre physiologisch verträglichen Salze und N-Oxide, Verfahren zu ihrer Herstellung und dafür verwendbare Zwischenprodukte sowie ihre Verwendung als Leistungsförderer für Tiere.

# Heteroarylethylamine, Verfahren zu ihrer Herstellung und ihre Verwendung als Leistungsförderer bei Tieren

Die vorliegende Erfindung betrifft neue Heteroarylethylamine, Verfahren zu ihrer Herstellung und ihre Verwendung als Leistungsförderer bei Tieren.

Heteroarylethylamine sind bereits bekannt. Sie besitzen beta-sympathomimetische Wirkung (DE-OS 2 603 600, EP-OS 120 770, US-P 4 358 955). Es ist jedoch nichts über die Eignung dieser Verbindungen als Leistungsförderer bei Tieren bekannt geworden.

Es wurden nun gefunden:

1. Neue Verbindungen der Formel I

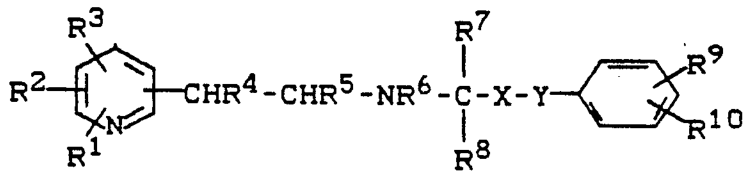

(I)

in welcher

$R^1$ für Wasserstoff, Alkyl, Halogen, Halogenalkyl, Hydroxy, Cyano, Alkoxycarbonyl, Aminocarbonyl, Mono- und Dialkylaminocarbonyl, Alkoxy, Halogenalkoxy, Halogenalkylthio, $NHSO_2$-Alkyl steht,

$R^2$ für Wasserstoff, Hydroxy, Alkoxy oder den Rest $-NR^{11}R^{12}$ steht,

$R^3$ für die bei $R^1$ angegebenen Reste steht,

$R^4$ für Hydroxy, Acyloxy oder Alkoxy steht,

$R^5$ für Wasserstoff oder Alkyl steht,

$R^6$ für Wasserstoff oder Alkyl steht,

$R^7$ für Wasserstoff oder Alkyl steht,

$R^8$ für Wasserstoff oder Alkyl steht,

X für $C_1$-$C_{10}$-Alkylen oder eine direkte Bindung steht,

Y für Sauerstoff oder eine direkte Bindung steht,

$R^9$ für Wasserstoff, $C_1$-$C_{10}$-Alkyl, welches gegebenenfalls durch Hydroxy, Alkoxy, Acyloxy oder den Rest $-NR^{13}R^{14}$ substituiert ist, sowie für den Rest $COR^{15}$ oder den Rest O-Z-$R^{16}$ steht,

Z für $C_1$-$C_{10}$-Alkylen, -Alkenylen oder -Alkinylen steht,

$R^{10}$ für Wasserstoff, Halogen, Alkyl, Alkoxy, Hydroxy oder den Rest $COR^{15}$ steht,

$R^{11}$ für Wasserstoff, oder Alkyl steht,

$R^{12}$ für Wasserstoff, Alkyl, Halogenalkyl oder Acyl steht,

$R^{13}$ für Wasserstoff, gegebenenfalls substituiertes Alkyl, gegebenenfalls substituiertes Aryl steht,

$R^{14}$ für die bei $R^{13}$ angegebenen Reste steht,

$R^{15}$ für Hydroxy, Alkoxy oder den Rest $NR^{13}R^{14}$ steht,

$R^{16}$ für Hydroxy, Alkoxy, Acyloxy, den Rest $NR^{13}R^{14}$ oder den Rest $COR^{15}$ steht,

sowie ihre physiologische verträglichen Salze und N-Oxide.

2. Verfahren zur Herstellung der Verbindungen der Formel I,

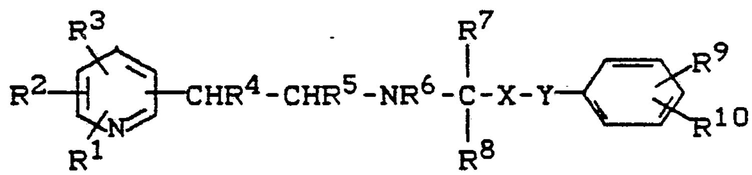

(I)

in welcher

$R^1$ bis $R^{10}$, X und Y die oben angegebene Bedeutung haben,

(a) indem man Halogenmethylketone der Formel II,

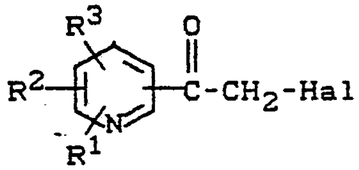

(II)

in welcher

$R^1$, $R^2$, $R^3$ die oben angegebene Bedeutung haben und Hal für Halogen steht,

mit Aminen der Formel III

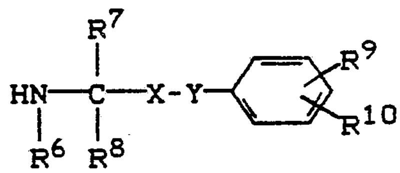

(III)

in welcher

$R^6$ bis $R^{10}$, X und Y die oben angegebene Bedeutung haben,

umsetzt und anschließend die Carbonylgruppe reduziert, oder

(b) indem man Epoxide der Formel IV

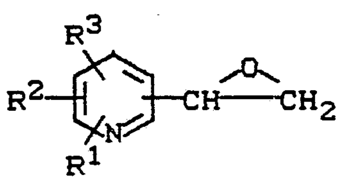

(IV)

in welcher

$R^1$, $R^2$, $R^3$ die oben angegebene Bedeutung haben,

mit Aminen der Formel III

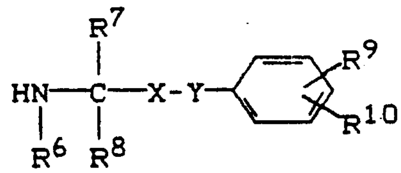

(III)

in welcher

$R^6$ bis $R^{10}$, X und Y die oben angegebene Bedeutung haben,

umsetzt, oder

(c) indem man ß-Halogenethylverbindungen der Formel V

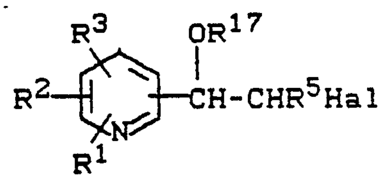

(V)

in welcher

$R^1$, $R^2$, $R^3$ und $R^5$ die oben angegebene Bedeutung haben,

Hal für Halogen steht,

$R^{17}$ für Wasserstoff oder $C_1$-$C_4$-Alkyl steht,

mit Aminen der Formel III

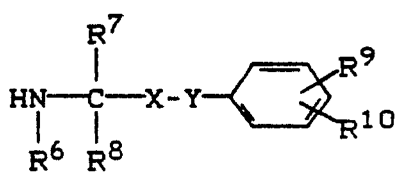

(III)

in welcher

$R^6$ bis $R^{10}$, X und Y die oben angegebene Bedeutung haben,

umsetzt, oder

d) indem man für den Fall, daß in Formel I $R^6$ und $R^7$ für Wasserstoff stehen,

Verbindungen der Formel VI

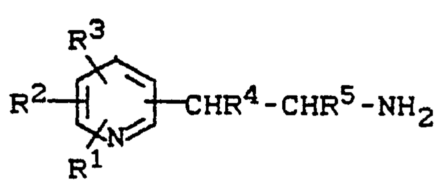

(VI)

in welcher

$R^1$ bis $R^5$ die oben angegebene Bedeutung haben,

mit Ketonen der Formel VII

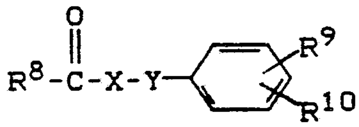

(VII)

in welcher

$R^8$, $R^9$, $R^{10}$, X und Y die oben angegebene Bedeutung haben,

unter reduzierenden Bedingungen umsetzt, oder

e) indem man, für den Fall daß in Formel I $R^4$ für Hydroxy steht und $R^5$ und $R^6$ für Wasserstoff stehen,

Verbindungen der Formel VIII

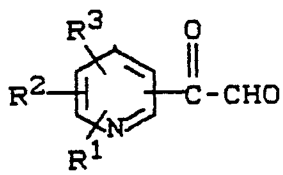

(VIII)

in welcher

$R^1$, $R^2$, $R^3$ die oben angegebene Bedeutung haben,

min Aminen der Formel IX

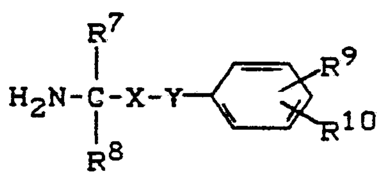

(IX)

in welcher

$R^7$ bis $R^{10}$ die oben angegebene Bedeutung haben, unter reduzierenden Bedingungen umsetzt, oder

f) indem man, für den Fall, daß in Formel I $R^4$ für Hydroxy und $R^6$ für Wasserstoff stehen,

Verbindungen der Formel X

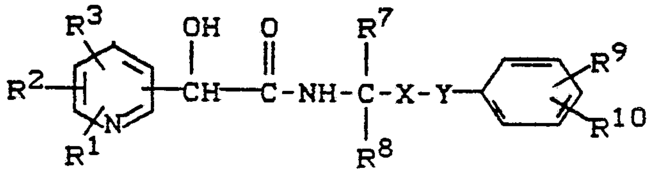

(X)

in welcher

$R^1$, $R^2$, $R^3$, $R^7$, $R^8$, $R^9$, $R^{10}$, X und Y die oben angegebene Bedeutung haben,

reduziert.

3. Neue Verbindungen der Formel X

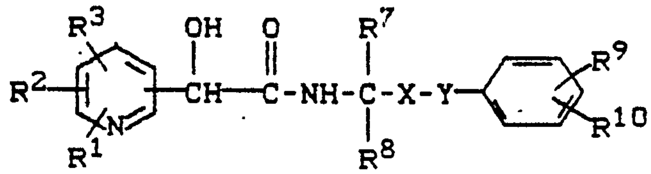

(X)

in welcher

$R^1$, $R^2$, $R^3$, $R^7$, $R^8$, $R^9$, $R^{10}$, X und Y die bei den Verbindungen der Formel I angegebenen Bedeutungen haben.

4. Verfahren zur Herstellung der Verbindungen der Formel X, dadurch gekennzeichnet, daß man Verbindungen der Formel XI

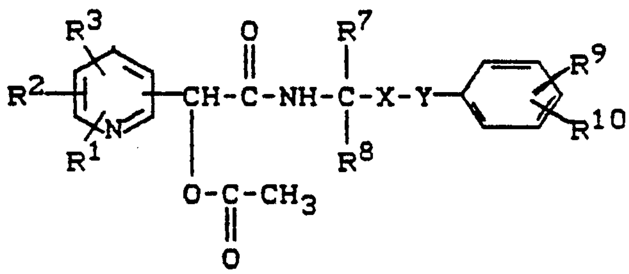

(XI)

in welcher

$R^1$ bis $R^{10}$, X und Y die oben angegebene Bedeutung haben,

hydrolysiert.

5. Neue Verbindungen der Formel XI

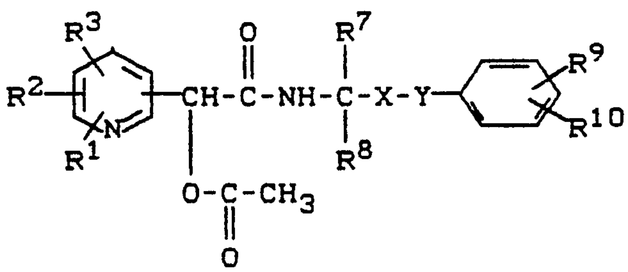

(XI)

in welcher

$R^1$ bis $R^{10}$, X und Y die oben angegebene Bedeutung haben.

6. Verfahren zur Herstellung der Verbindungen der Formel XI, dadurch gekennzeichnet, daß man Aldehyde der Formel XII

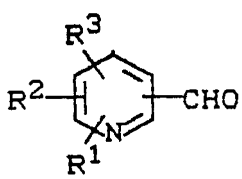

(XII)

in welcher

$R^1$, $R^2$, $R^3$ die oben angegebene Bedeutung haben,

mit Isonitrilen der Formel XIII

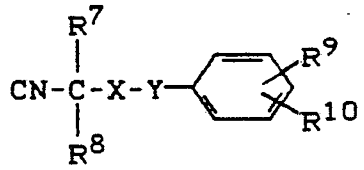

(XIII)

in welcher

$R^7$ bis $R^{10}$, X und Y die oben angegebene Bedeutung haben,

in Gegenwart von Essigsäure umsetzt.

Die Verbindungen der Formal I können in Form ihrer Tautomeren vorliegen. Beispiele dafür sind für den Fall, daß
$R^2$ für $NH_2$ steht:

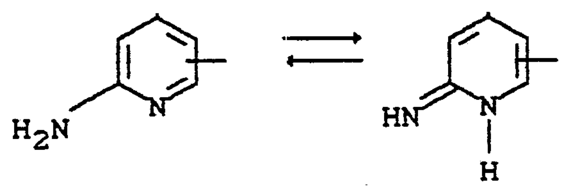

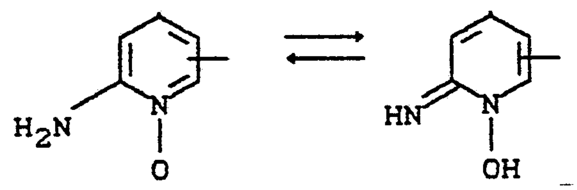

Die Verbindungen der Formel I können auch in Form ihrer sterischen und optischen Isomeren vorliegen und dabei zueinander enantiomere und/oder diastereomere Formen bilden.

Physiologisch verträgliche Salze der Verbindungen der Formel I können mit folgenden Säuren gebildet werden:
Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbundungen der Formel I

in welcher

$R^1$ für Wasserstoff, $C_{1-4}$-Alkyl, Fluor, Chlor, Brom, $C_{1-4}$-Halogenalkyl, Hydroxy, Cyano, $C_{1-4}$-Alkoxycarbonyl, Mono-oder -di-$C_{1-4}$-alkylaminocarbonyl, $C_{1-6}$-Alkoxy, $C_{1-6}$-Halogenalkoxy, $C_{1-6}$-Halogenalkylthio, -$NHSO_2$-C $_{1-6}$-Alkyl steht,

$R_2$ für Wasserstoff, Hydroxy, $C_{1-6}$-Alkoxy oder den Rest -$NR^{11}R^{12}$ steht,

$R^3$ für die bei $R^1$ angegebenen Reste steht,

$R^4$ für OH, $C_{1-6}$-Alkoxy oder Acyloxy steht und Acylox für Oxycarbonyl-C $_{1-6}$-alkyl, gegebenenfalls

substituiertes Oxycarbonylphenyl, Oxysulfonyl-$C_{1-6}$-alkyl, gegebenenfalls substituiertes Oxysulfonylphenyl steht,

$R^5$ für Wasserstoff oder $C_1$-$C_6$-Alkyl steht,

$R^6$ für Wasserstoff, gegebenenfalls substituiertes $C_{1-6}$-Alkyl steht,

$R^7$ für Wasserstoff oder $C_1$-$C_6$-Alkyl steht,

$R^8$ für Wasserstoff oder $C_1$-$C_6$-Alkyl steht,

X für $C_1$-$C_6$-Alkylen oder eine direkte Bindung steht,

Y für Sauerstoff oder eine direkte Bindung steht,

$R^9$ für Wasserstoff, $C_1$-$C_6$-Alkyl, welches gegebenenfalls durch Hydroxy, $C_1$-$C_6$-Alkoxy, $C_1$-$C_6$-Acyloxy oder den Rest $NR^{13}R^{14}$ substiutiert ist, sowie für den Rest $COR^{15}$ oder den Rest O-Z-$R^{16}$ steht,

Z für $C_1$-$C_6$-Alkylen, $C_2$-$C_6$-Alkenylen oder $C_2$-$C_6$-Alkinylen steht,

$R^{10}$ für Wasserstoff, Halogen, $C_1$-$C_6$-Alkyl, $C_1$-$C_6$-Alkoxy, Hydroxy oder den Rest $COR^{15}$ steht,

$R^{11}$ für Wasserstoff oder $C_1$-$C_6$-Alkyl steht,

$R^{12}$ für Wasserstoff, $C_1$-$C_6$-Alkyl, $C_{1-4}$ -Halogenalkyl, $C_{1-6}$-Alkylcarbonyl, gegebenenfalls substituiertes Phenylsulfonyl steht,

$R^{13}$ für Wasserstoff, $C_1$-$C_6$-Alkyl, welches gegebenenfalls substituiert ist, steht,

$R^{14}$ für die bei $R^{13}$ angegebenen Reste steht,

$R^{15}$ für Hydroxy, $C_1$-$C_6$-Alkoxy oder den Rest $NR^{13}R^{14}$ steht,

$R^{16}$ für Hydroxy, $C_1$-$C_6$-Alkoxy, $C_1$-$C_6$-Acyloxy, den Rest $NR^{13}R^{14}$ oder den Rest $COR^{15}$ steht.

Als Substituenten der gegebenenfalls substituierten Reste kommen bevorzugt infrage: Cyano, Halogen wie Fluor oder Chlor, Hydroxy, $C_{1-4}$ -Alkyl, $C_{1-4}$-Halogenalkyl, Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich bevorzugt Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können.

Besonders bevorzugt sind Verbindungen der Formel I

in welcher

$R^1$ für Wasserstoff, $C_{1-4}$-Alkyl, Halogen, insbesondere Fluor, Chlor, Brom, Cyano, Hydroxy, $C_{1-4}$-Halogenalkyl mit 1 bis 5 Halogenatomen, $C_{1-4}$-Alkoxy, $C_{1-4}$-Halogenalkoxy mit 1 bis 5 Halogenatomen, $NHSO_2$-$C_{1-4}$-Alkyl, -COO-$C_{1-4}$-Alkyl, -$CONH_2$, -CONH-$C_{1-4}$-Alkyl, -CON($C_{1-4}$-Alkyl) steht,

$R^2$ für Wasserstoff, OH, $C_{1-4}$-Alkoxy, -$NR^{11}R^{12}$ steht,

$R^3$ für die bei $R^1$ angegebenen Reste steht,

$R^4$ für OH, $C_{1-6}$-Acyloxy, insbesondere Acetoxy steht,

$R^5$ für Wasserstoff oder $C_1$-$C_3$-Alkyl, insbesondere Methyl oder Ethyl steht,

$R^6$ für Wasserstoff steht,

$R^7$ für Wasserstoff steht,

$R^8$ für Wasserstoff oder $C_1$-$C_3$-Alkyl, insbesondere Methyl oder Ethyl steht,

X für $C_1$-$C_3$-Alkylen, insbesondere Methylen oder Ethylen steht,

Y für eine direkte Bindung steht,

$R^9$ für Wasserstoff, $C_1$-$C_3$-Alkyl, welches bevorzugt durch Hydroxy, $C_1$-$C_3$-Alkoxy, insbesondere Methoxy, $C_1$-$C_3$-Acyloxy, insbesondere Acetoxy oder den Rest $NR^{13}R^{14}$ substituiert ist, für den Rest $COR^{15}$ oder den Rest O-Z-$R^{16}$ steht,

Z für $C_1$-$C_4$-Alkylen, insbesondere Methylen oder Ethylen, $C_2$-$C_4$-Alkenylen oder $C_2$-$C_4$-Alkinylen steht,

$R^{10}$ für Wasserstoff, Halogen, insbesondere Fluor, Chlor, Brom, $C_1$-$C_3$-Alkyl, insbesondere Methyl, $C_1$-$C_3$-Alkoxy, insbesondere Methoxy, Hydroxy oder den Rest $COR^{15}$ steht,

$R^{11}$ für Wasserstoff oder $C_1$-$C_3$-Alkyl, insbesondere Methyl oder Ethyl steht,

$R^{12}$ für Wasserstoff oder Methyl steht,

$R^{13}$ für Wasserstoff, $C_1$-$C_3$-Alkyl, insbesondere Methyl oder Ethyl steht,

$R^{14}$ für Wasserstoff steht,

$R^{15}$ für Hydroxy, $C_1$-$C_3$-Alkoxy, insbesondere Methoxy oder Ethoxy, oder den Rest $NR^{13}R^{14}$ steht,

$R^{16}$ für Hydroxy, $C_1$-$C_3$-Alkoxy, insbesondere Methoxy, $C_1$-$C_3$-Acyloxy, insbesondere Acetoxy, den Rest $NR^{13}R^{14}$ oder den Rest $COR^{15}$ steht.

Im einzelnen seien neben den Beispielen die folgenden Verbindungen der Formel I genannt:

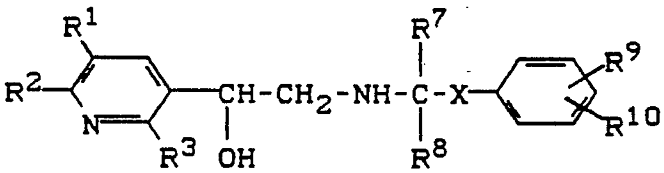

| $R^1$ | $R^2$ | $R^3$ | $R^7$ | $R^8$ | X | $R^9$ | $R^{10}$ |
|---|---|---|---|---|---|---|---|
| Cl | $NH_2$ | H | H | $CH_3$ | $-CH_2-$ | H | 3-Cl |
| CN | $NH_2$ | H | H | H | $-CH_2-$ | $4-CH_3$ | H |
| Cl | OH | H | $CH_3$ | H | $-(CH_2)_2-$ | $4-COOCH_3$ | 3-Cl |
| CN | OH | H | $C_2H_5$ | H | $-(CH_2)_2-$ | $4-OCH_2-COOCH_3$ | 2-Br |
| H | $NH_2$ | H | $CH_3$ | H | $-CH_2-$ | $4-OCH_2-COOCH_3$ | H |
| H | $NH_2$ | H | H | H | $-CH_2-$ | H | $4-COOCH_3$ |

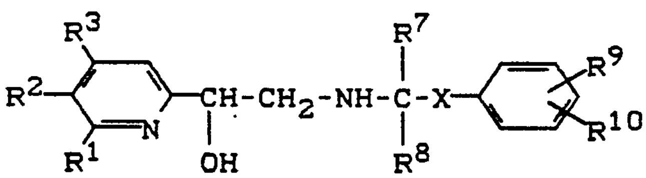

| $R^1$ | $R^2$ | $R^3$ | $R^7$ | $R^8$ | X | $R^9$ | $R^{10}$ |
|---|---|---|---|---|---|---|---|
| Cl | $NH_2$ | Cl | H | H | $-CH_2-$ | $4-OCH_2CH_2OH$ | $3-CH_3$ |
| Cl | $NH_2$ | Cl | $CH_3$ | H | $-(CH_2)_2-$ | $4-OCH_2COOCH_3$ | H |
| Cl | $NH_2$ | Cl | H | $C_2H_5$ | $-CH_2$ | $4-OCH_2COOH$ | 2-Cl |
| Cl | $NH_2$ | Cl | $C_2H_5$ | $CH_3$ | $-(CH_3)_3-$ | $4-COOC_2H_5$ | H |
| Cl | $NH_2$ | CN | $CH_3$ | H | $-CH_2-$ | $4-OCH_2CH_2NH_2$ | H |
| CN | $NH_2$ | Cl | H | $CH_3$ | $-(CH_2)_2-$ | $4-COOCH_3$ | H |
| Cl | $NH_2$ | CN | H | H | $-CH_2-$ | $4-OCH_2COOCH_3$ | 2-Br |
| CN | $NH_2$ | H | H | $CH_2$ | $-CH_2-$ | $4-OCH_2COOCH_3$ | H |
| H | $NH_2$ | CN | $CH_3$ | H | $-(CH_2)_2$ | $4-COOCH_3$ | 3-Cl |
| Br | $NH_2$ | CN | H | $CH_3$ | $-CH_2-$ | $4-OCH_2COOH$ | H |

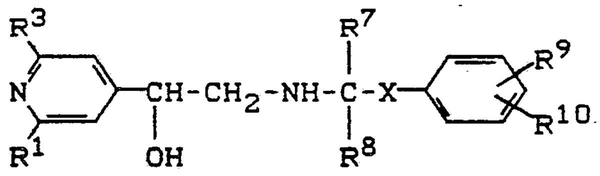

| $R^1$ | $R^3$ | $R^7$ | $R^8$ | X | $R^9$ | $R^{10}$ |
|---|---|---|---|---|---|---|
| Cl | Cl | H | $CH_3$ | $-CH_2-$ | $4-OCH_2COOCH_3$ | H |
| Cl | Cl | $CH_3$ | H | $-CH_2-$ | $4-OCH_2COOH$ | H |
| CN | H | H | $CH_3$ | $-CH_2-$ | $4-OCH_2COOCH_3$ | H |
| Cl | H | H | $C_2H_5$ | $-(CH_2)_2-$ | $4-OCH_2CH_2OH$ | 3-Cl |

Bevorzugt seien die Salze mit Salzsäure, Schwefelsäure, Phosphorsäure, Oxalsäure, Maleinsäure, Fumarsäure, Malonsäure genannt.

Die Verbindungen der Formel I lassen sich nach den oben unter 2. angegebenen Verfahren a) bis f) herstellen.

Setzt man bei Verfahren 2a) als Halogenmethylketon der Formel II 2-Chloracetyl-6-methoxypyridin und als Amin der Formel III 2-(4-Methoxycarbonylphenyl)ethylamin ein, läßt sich Verfahren a) durch folgendes Reaktionsschema wiedergeben:

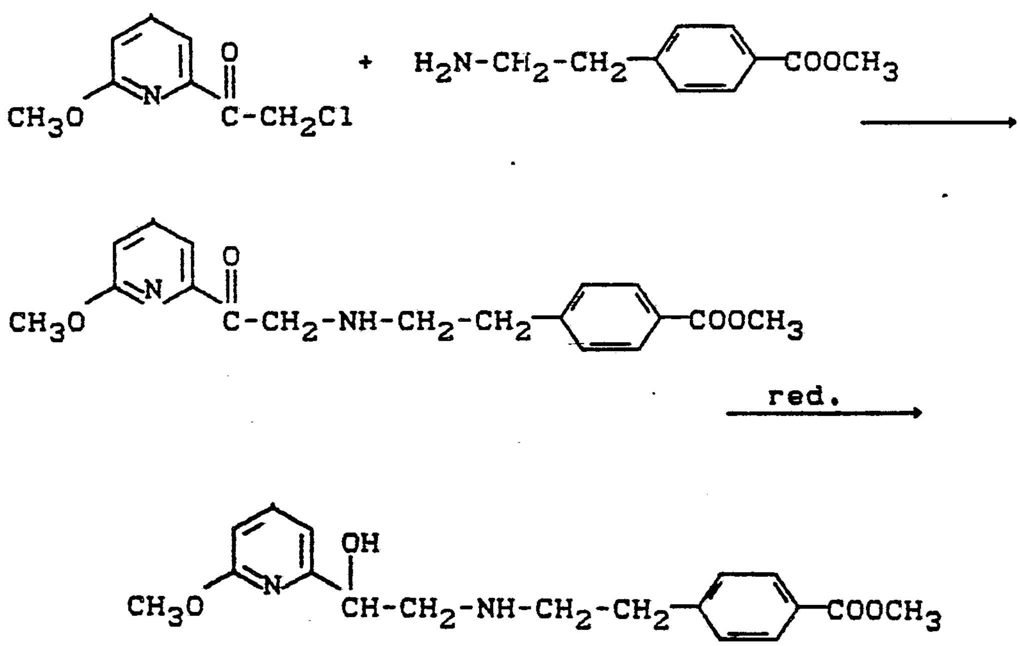


Die Verbindungen der Formel II sind Gegenstand einer älteren nicht vorveröffentlichten Patentanmeldung der Anmelderin, Deutsche Patentanmeldung P 3 615 293d.5.

Sie werden hergestellt, indem man in an sich bekannter Weise die entsprechenden acetylsubstituierten Heteroarylverbindungen mit elementarem Halogen oder mit Kupferhalogeniden umsetzt. Die acetylsubstituierten Heteroarylverbindungen sind bekannt aus C.T. Gnewuch J. Med. Chem. 15, 1321 (1972), US-P 4 358 455 oder lasen sich nach den dort angegebenen Verfahren herstellen.

Die Substiuienten $R^1$, $R^2$ und $R^3$ in Formel II besitzen bevorzugt die weiter oben bei den Verbindungen der Formel I angegebenen bevorzugten Bedeutungen. Im einzelnen seien folgende Verbindungen der Formel II genannt:

(2-Amino-3-chlor-5-pyridyl)-chlormethylketon,
(2-Amino-3-chlor-5-pyridyl)-chlormethylketon,
(2,4-Dichlor-3-amino-6-pyridyl)-brommethylketon,
(2-Cyano-3-amino-6-pyridyl)-brommethylketon,
(3-Amino-4-cyano-6-pyridyl)-brommethylketon,
(3-Amino-4-cyano-6-pyridyl)-chlormethylketon,
(2-Cyano-3-amino-4-chlor-6-pyridyl)-brommethylketon,
(2-Cyano-3-amino-4-chlor-6-pyridyl)-chlormethylketon,
(2-Chlor-3-amino-4-trifluormethyl-6-pyridyl)-brommethylketon,
(2-Trifluormethyl-3-amino-4-cyano-6-pyridyl)-brommethylketon,
(2-Fluor-3-amino-4-cyano-6-pyridyl)-chlormethylketon.

Die Amine der Formel III sind bekannt (vgl. z.B. EP-OS 70 133) oder lassen sich analog zu bekannten Verfahren herstellen. Die Substituenten $R^6$ bis $R^{10}$ sowie X und Y besitzen bevorzugt die weiter oben bei den Verbindungen der Formel I angegebenen bevorzugten Bedeutungen. Im einzelnen seien folgende Verbindungen der Formel III genannt:

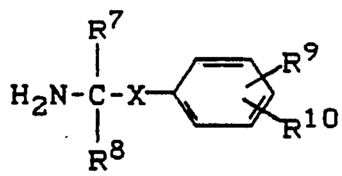

| $R^7$ | $R^8$ | X | $R^9$ | $R^{10}$ |
|---|---|---|---|---|
| H | $CH_3$ | $-CH_2-$ | $4-OCH_2COOCH_3$ | H |
| $CH_3$ | H | $-CH_2-$ | $4-OCH_2COOH$ | H |
| H | $CH_3$ | $-CH_2-$ | $4-OCH_2CH_2OH$ | $3-CH_3$ |
| H | H | $-(CH_2)_2-$ | $4-OCH_2COOC_2H_5$ | 2-Cl |

Als Reduktionsmittel zur Durchführung des Verfahrens 2a) seien folgende Reduktionsmittel genannt:
$H_2$/Katalysator, als Katalysator seien beispielsweise genannt: $PtO_2$, Pd-Aktivkohle;
komplexe Metallhydride wie z.B. $LiAlH_4$, $NaBH_4$, $NaBH_3CN$.

Besonders bevorzugt werden folgende Reduktionsmittel eingesetzt:

$NaBH_4$ und $NaBH_3CN$

Verfahren 2a) wird durchgeführt indem man Verbindungen II und III in einem Verdünnungsmittel in etwa äquimolaren Verhältnis zusammengibt.

Die Reaktion wird bei Temperaturen von -20°C bis +100°C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n-und i-Propanol.

Bevorzugt sind Alkohole, wobei ohne Isolierung der Zwischenstufen sofort die Reduktion durchgeführt werden kann.

Setzt man bei Verfahren 2b) als Epoxid der Formel IV 5-Methoxypyridin-3-epoxid und als Amin der Formel III 3-(4-Ethoxycarbonylmethoxyphenyl)-2-aminopropan ein, läßt sich Verfahren 2b) durch folgendes Reaktionsschema wiedergeben:

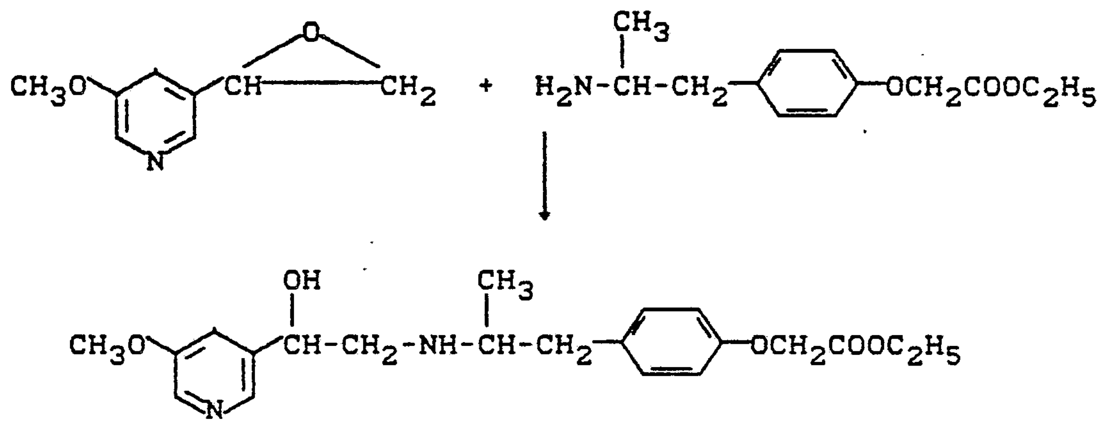

Epoxide der Formel IV sind Gegenstand einer älteren nicht vorveröffentlichten Patentanmeldung der Anmelderin, Deutsche Patentanmeldung P 3 615 293.5. Sie werden hergestellt, indem man in an sich bekannter Weise Halogenmethylverbindungen der Formel

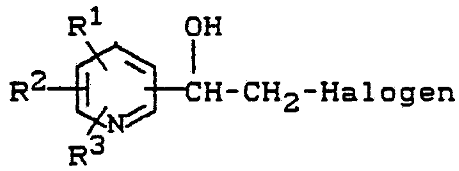

in welcher

$R^1$, $R^2$ und $R^3$ die bei den Verbindungen der Formel I angegebenen Bedeutungen besitzen,

mit Basen umsetzt oder die entsprechenden Aldehyde der Formel

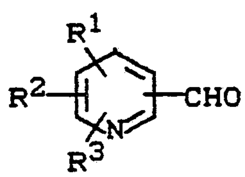

mit Methylgruppen-übertragenden Reagenzien in Gegenwart von Basen unter den Bedingungen der Corey-Epoxidierung (E. J. Corey, J.A.C.S. 87, 1353 (1955)) umsetzt.

Die Aldehyde werden erhalten, indem man die entsprechenden Alkohole der Formel

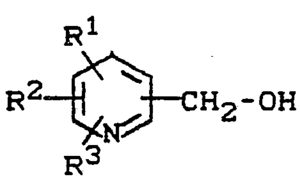

oxidiert oder die entsprechenden Säurechloride der Formel

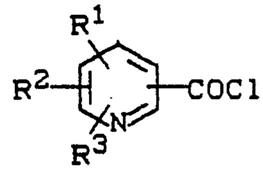

reduziert. Die Alkohole und Säurechloride sind bekannt.

Im einzelnen seien die folgenden Epoxide genannt:

2-Amino-3-chlorpyridin-5-epoxid,
2-Amino-3-cyanopyridin-5-epoxid,
2,4-Dichlor-3-aminopyridin-6-epoxid,
2-Chlor-3-amino-4-cyanopyridin-6-epoxid,
2-Cyano-3-amino-4-chlorpyridin-6-epoxid,
2-Cyano-3-aminopyridin-6-epoxid,
2-Chlor-3-amino-4-trifluormethylpyridin-6-epoxid,
2-Brom-3-amino-4-cyanopyridin-6-epoxid.

Verfahren b) wird durchgeführt indem man etwa äquimolare Mengen des Epoxids der Formel IV und des Amins der Formel III in einem Verdünnungsmittel umsetzt.

Im allgemeinen wird ein Überschuß an Amin (1-3 molar, bevorzugt 1-1,5 molar) bezogen auf das Epoxid der Formel IV verwendet.

Die Reaktion wird bei Temperaturen von +20 bis +150°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, ferner Nitrile wie Acetonitril und Benzonitril, Amide wie Dimethylformamid, Alkohole wie Methanol, Ethanol, n-und i-Propanol.

Bevorzugt sind Alkohole.

Setzt man bei Verfahren 2c) als ß-Halogenmethylverbindung der Formel V 5-Methoxy-3-(1-hydroxy-2-chlorethyl)pyridin und als Amin der Formel III 2-(4-Methoxyphenyl)-1-methylethylamin ein, läßt sich Verfahren c) durch folgendes Formelschema wiedergeben:

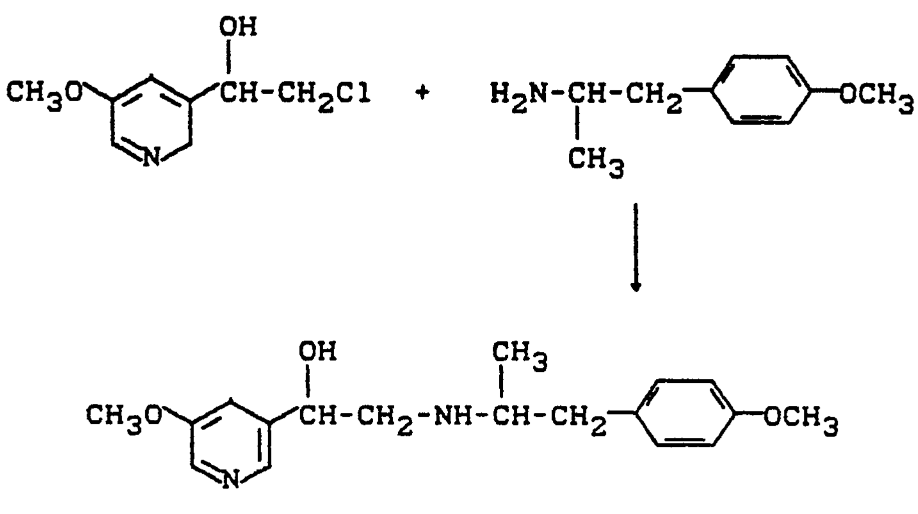

ß-Halogenmethylverbindungen der Formel V sind Gegenstand einer älteren nicht vorveröffentlichten Patentanmeldung der Anmelderin (Deutsche Patentanmeldung P 3 615 293.5) und werden erhalten, indem man die entsprechenden Halogenmethylketone reduziert oder die entsprechenden Heteroarylvinylverbindungen mit N-Halogenacetamiden umsetzt.

Im einzelnen seien folgende Verbindungen der Formel V genannt:
1-(2-Amino-3-chlor-5-pyridyl)-2-chlorethanol,
1-(2-Amino-3-cyano-5-pyridyl)-2-chlorethanol,
1-(2,4-Dichlor-3-amino-6-pyridyl)-2-chlorethanol,
1-(2-Chlor-3-amino-4-cyano-6-pyridyl)-2-chlorethanol,
1-(2-Cyano-3-amino-4-chlor-6-pyridyl)-2-bromethanol,
1-(2-Cyano-3-amino-6-pyridyl)-2-chlorethanol,
1-(3-Amino-4-cyano-6-pyridyl)-2-bromethanol,
1-(2-Chlor-3-amino-4-trifluormethyl-6-pyridyl)-2-chlorethanol,
1-(2-Cyano-3-amino-4-fluor-6-pyridyl)-2-bromethanol.

Verfahren 2c) wird durchgeführt indem man die beta-Halogenmethylverbindung der Formel V mit überschüssigem Amin der Formel III gegebenenfalls in Gegenwart eines Verdünnungsmittels umsetzt.

Die Reaktion wird bei Temperaturen von +20 bis +150°C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n-und i-Propanol.

Bevorzugt eingesetzt werden Alkohole.

Setzt man bei Verfahren 2d) als Verbindung der Formel VI 5-Fluor-3-(1-hydroxy-2-aminoethyl)-pyridin und als Verbindung der Formel VII (3-Chlor-4-methoxyphenyl)-aceton ein, läßt sich Verfahren d) durch folgendes Formelschema wiedergeben:

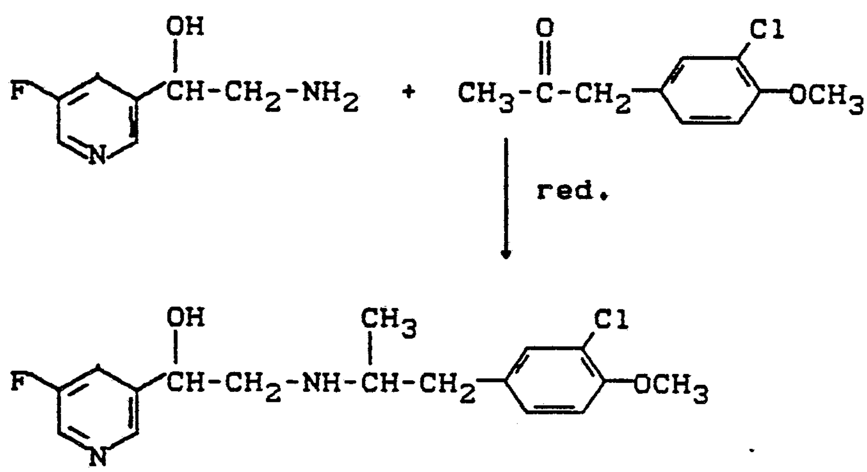


Verbindungen der Formel VI sind Gegenstand einer älteren nicht vorveröffentlichten Patentanmeldung der Anmelderin (Deutsche Patentanmeldung P 3 615 293.5) und lassen sich herstellen, indem man die entsprechenden Nitroverbindungen reduziert. Die Nitroverbindungen sind bekannt (K.W. Merz Arch. Pharm. 1964, 10) oder lassen sich analog zu bekannten Verfahren herstellen.

Im einzelnen seien folgende Verbindungen der Formel VI genannt:

1-(2-Amino-3-chlor-5-pyridyl)-2-aminoethanol,
1-(2-Amino-3-cyano-5-pyridyl)-2-aminoethanol,
1-(2,4-Dichlor-3-amino-6-pyridyl)-2-aminoethanol,
1-(2-Chlor-3-amino-4-cyano-6-pyridyl)-2-aminoethanol,
1-(2-Cyano-3-amino-6-pyridyl)-2-aminoethanol,
1-(2-Chlor-3-amino-4-trifluormethyl-6-pyridyl)-2-aminoethanol.

Sie lassen sich in den Fällen, in denen in Formel VI die Reste $R^1$, $R^2$ und $R^3$ nicht gleichzeitig für Wasserstoff stehen und $R^4$ für OH steht, herstellen, indem man Verbindungen der Formel XIV

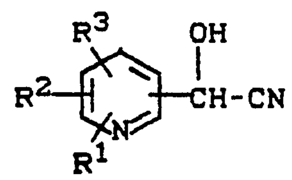

(XIV)

in welcher

$R^1$, $R^2$ und $R^3$ die bei den Verbindungen der Formel I angegebenen Bedeutungen haben, unter der Voraussetzung, daß nicht alle Reste $R^1$, $R^2$ und $R^3$ gleichzeitig für Wasserstoff stehen dürfen,

reduziert.

Verbindungen der Formel XIV sind neu. Sie werden erhalten, indem man Aldehyde der Formel XII

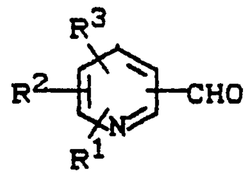

(XII)

in welcher

$R^1$, $R^2$ und $R^3$ die bei den Verbindungen der Formel I angegebene Bedeutung haben, unter der Voraussetzung, daß nicht alle Reste $R^1$, $R^2$, $R^3$ gleichzeitig für Wasserstoff stehen dürfen,

mit Cyanwasserstoff, ihren Salzen oder mit Cyanhydrinen niederer aliphatischer Ketone umsetzt.

Setzt man bei der Herstellung der Verbindungen der Formel VI als Verbindung der Formel XIV 2-Chlor-4-pyridylcyanhydrin ein, läßt sich das Verfahren durch das folgende Schema darstellen:

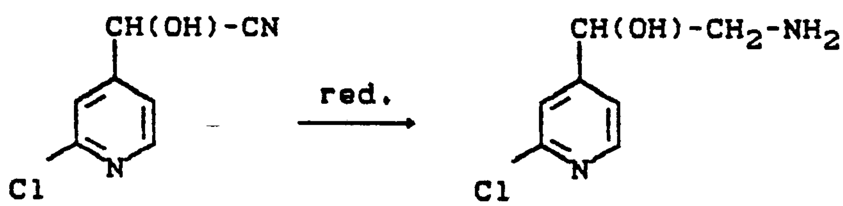


Die Verbindungen der Formel XIV sind neu. Ihre Herstellung wird weiter unten beschrieben. Die Substituenten $R^1$, $R^2$ und $R^3$ in den Verbindungen XIV besitzen die bei den Verbindungen der Formel I angegebenen bevorzugten Bedeutungen, wobei $R^1$, $R^2$ und $R^3$ nicht gleichzeitig für Wasserstoff stehen dürfen. Im einzelnen seien folgende Verbindungen der Formel XIV genannt:

(2,6-Dichlor-4-pyridyl)-cyanhydrin
(2-Amino-5-pyridyl)-cyanhydrin
(2-Amino-3-chlor-5-pyridyl)-cyanhydrin
3(-Amino-6-pyridyl)-cyanhydrin
(2-Chlor-3-amino-6-pyridyl)-cyanhydrin
(2,4-Dichlor-3-amino-6-pyridyl)-cyanhydrin.

Das Verfahren wird durchgeführt, indem man die Verbindung XIV in einem Verdünnungsmittel reduziert.

Die Reaktion wird bei Temperaturen von 0°C bis 150°C durchgeführt.

Es wird bei Normaldruck oder bei erhöhtem Druck gearbeitet.

Als Verdünnungsmittel dienen, abhängig von Reduktionsmittel, Wasser oder organische Lösungsmittel oder Mischungen hiervon. Zu den organischen Lösungsmitteln gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen:

$H_2$/Katalysator, als Katalysator sei beispielsweise $PtO_2$ genannt; Alkali-und Erdalkaliamalgame wie z.B. Natriumamalgam; unedle Metalle in Gegenwart von Salzsäure wie z. B. Zink/Salzsäure; komplexe Metallhydride wie z. B. $LiAlH_4$; Borane wie z. B. Diboran.

Wie bereits erwähnt, sind die Verbindungen der Formel XIV neu.

Setzt man zu ihrer Herstellung als Verbindung der For mel XII 2-Fluorpyridin-5-aldehyd ein, läßt sich das Verfahren durch das folgende Scheme wiedergeben:

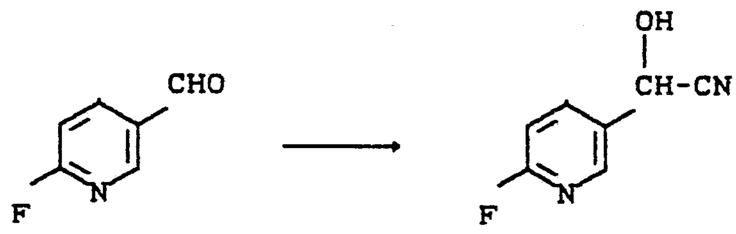

Die Herstellung der Aldehyde der Formel XII ist bereits bei Verfahren 2) (oben) beschrieben. Die Substituenten $R^1$, $R^2$ und $R^3$ in Formel XII besitzen die bei den Verbindungen der Formel I angegebenen Bedeutungen, wobei $R^1$, $R^2$ und $R^3$ nicht gleichzeitig für Wasserstoff stehen dürfen. Im einzelnen seien folgende Verbindungen der Formel XII genannt:

2,6-Dichlorpyridin-4-aldehyd
2-Aminopyridin-5-aldehyd
2-Amino-3-chlorpyridin-5-aldehyd
3-Aminopyridin-6-aldehyd
2-Chlor-3-aminopyridin-6-aldehyd
2,4-Dichlor-3-aminopyridin-6-aldehyd.

Das Verfahren wird durchgeführt, indem man in literaturbekannter Weise die Aldehyde der Formel XII bzw. deren Hydrogensulfitadditionsprodukte mit Cyanwasserstoff oder ihren Salzen oder niederaliphatischen Ketoncyanhydrinen umsetzt (P. Kurtz, in Houben-Weyl, Bd. VIII, S. 274 ff).

Verbindungen der Formel VII sind bekannt (vgl. z.B. EP-OS 23 385) oder lassen sich analog zu bekannten Verbindungen herstellen. Die Substituenten $R^8$ bis $R^{10}$ sowie X und Y besitzen die weiter oben angegebenen Bedeutungen.

Im einzelnen seien folgende Verbindungen der Formel VII genannt:

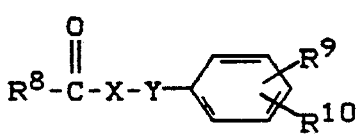

| $R^8$ | X | Y | $R^9$ | $R^{10}$ |
|---|---|---|---|---|
| H | $-CH_2-$ | - | $4-COOCH_3$ | H |
| $CH_3$ | $-CH_2-$ | - | $4-OCH_2COOCH_3$ | H |
| $CH_3$ | $-CH_2-$ | - | $4-OCH_2CH_2OH$ | 3-Cl |
| $CH_3$ | $-(CH_2)_2-$ | O | 4-COOH | $2-CH_3$ |
| H | $-CH_2-$ | O | $4-OCH_2COOH$ | H |

Verfahren 2d) wird durchgeführt, indem man etwa äquimolare Mengen der Verbindungen der Formeln VI und VII in einem Verdünnungsmittel vorlegt und die Mischung reduziert.

Die Reaktion wird bei Temperaturen von 0°C bis 150°C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen:

$H_2$/Katalysator, als Katalysator sei beispielsweise $PtO_2$ genannt; komplexe Metallhydride wie z.B. $LiAlH_4$, $NaBH_4$, $NaBH_3CN$

Setzt man bei Verfahren 2e) als Verbindung der Formel VIII 5-Methyl-3-pyridylglyoxal und als Amin der Formel IX 2-(3-Brom-4-methoxyphenyl)-ethylamin ein, läßt sich Verfahren e) durch folgendes Reaktionsschema wiedergeben:

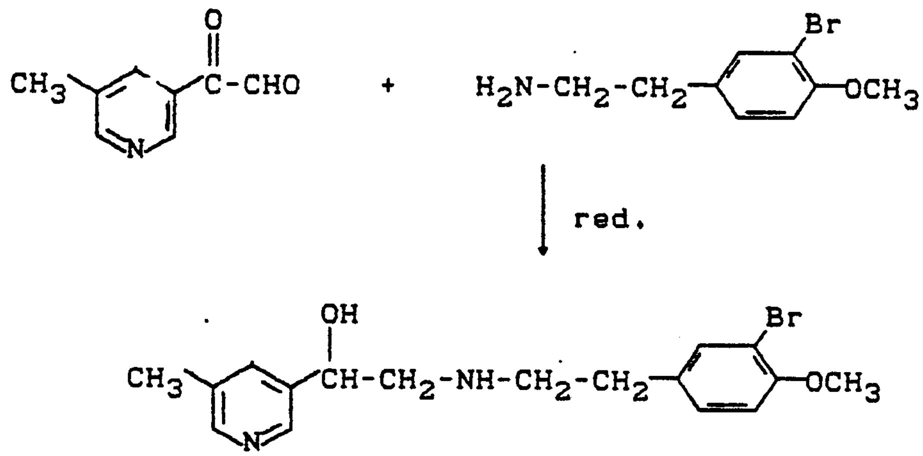


Die Herstellung der Verbindungen der Formel VIII ist bereits bei Verfahren 2b erwähnt. Verbindungen der Formel IX sind bekannt (vgl. z.B. EP-OS 70 133) oder lassen sich analog zu bekannten Verbindungen herstellen.

Die Substituenten $R^1$, $R^2$, $R^3$ in Formel VIII besitzen bevorzugt die weiter oben bei den Verbindungen der Formel I angegebenen bevorzugten Bedeutungen. Im einzelnen seien folgende Verbindungen der Formel VIII genannt:

2-Amino-3-chlor-5-pyridylglyoxal,
2-Amino-3-cyano-5-pyridylglyoxal,
2,4-Dichlor-3-amino-6-pyridylglyoxal,
2-Cyano-3-amino-6-pyridylglyoxal,
2-Chlor-3-amino-4-trifluormethyl-6-pyridylglyoxal,

Verfahren 2e) wird durchgeführt, indem man zur Verbindung der Formel VIII in einem Verdünnungsmittel etwa die äquivalente Menge des Amins der Formel IX zusetzt und anschließend reduziert.

Die Reaktion wird bei Temperaturen von 0°C bis 100°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Di-glykoldimethylether, Tetrahydrofuran und Dioxan, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Tetramethylensulfon und Hexamethylphosphorsäuretriamid, weiterhin Alkohole wie Methanol, ethanol,, n-und i-Propanol.

Als Reduktionsmittel dienen

$H_2$/Katalysator; als Katalysator seien $PtO_2$ und Pd-Kohle genannt, ferner komplexe Metallhydride wie $LiAlH_4$ und $NaBH_4$.

Setzt man bei Verfahren 2f) als Verbindung der Formel X (5-Chlor-3-pyridyl)hydroxyessigsäure-2(4-methylphenyl)ethyl-amid ein, läßt sich das Verfahren durch folgendes Formelschema wiedergeben:

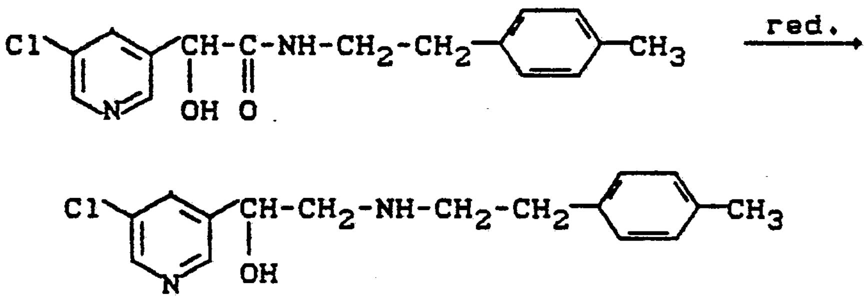


Verbindungen der Formel X sind neu. Ihre Herstellung wird weiter unten beschrieben. Die Substituenten $R^1$ bis $R^{10}$, X und Y in den Verbindungen X besitzen die bei den Verbindungen der Formel I angegebenen bevorzugten Bedeutungen. Im einzelnen seien folgende Verbindungen der Formel X genannt:

(2-Amino-5-pyridyl)hydroxyessigsäure-(3-(4-methoxycarbonyl-methoxyphenyl)-2-propyl)amid
(2-Amino-3-chlor-5-pyridyl)hydroxyessigsäure-(3-(4-ethoxycarbonylphenyl)-2-propyl)amid
(2-Amino-3-cyano-5-pyridyl)hydroxyessigsäure-(3-(4-methoxycarbonylmethoxyphenyl)-2-propyl)amid
(3-Amino-2,4-dichlor-6-pyridyl)hydroxyessigsäure-(3-(4-ethoxycarbonylmethoxyphenyl)-2-propyl)amid
(2-Cyano-3-amino-6-pyridyl)hydroxyessigsäure-(3-(4-methoxycarbonylphenyl)-2-propyl)amid
(3-Amino-4-cyano-6-pyridyl)hydroxyessigsäure-(3-(4-ethoxycarbonylmethoxyphenyl)-2-propyl)amid
(2,6-Dichlor-4-pyridyl)hydroxyessigsäure-(3-(4-methoxycarbonylphenyl)-2-propyl)amid
(2,6-Dichlor-4-pyridyl)hydroxyessigsäure-(3-(4-methoxycarbonylmethoxyphenyl)-2-propyl)amid.

Verfahren 2f) wird durchgeführt, indem man die Verbindung der Formel X in einem Verdünnungsmittel mit überschüssigem Reduktionsmittel umsetzt.

Die Reaktion wird bei Temperaturen von 0°C bis +150°C durchgeführt.

Es wird bevorzugt bei Normaldruck gearbeitet.

Als Verdünnungsmittel dienen alle inerten organischen Lösungsmittel, Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan.

Als Reduktionsmittel dienen komplexe Metallhydride wie $LiAlH_4$, Borane wie Diboran.

Setzt man bei dem Verfahren 4) als Verbindung der Formel XI (2-Cyano-5-pyridyl)-acetoxyessigsäure-(2-(3-chlor-4-methylphenyl)ethylamid ein, läßt sich das Verfahren durch das folgende Schema darstellen:

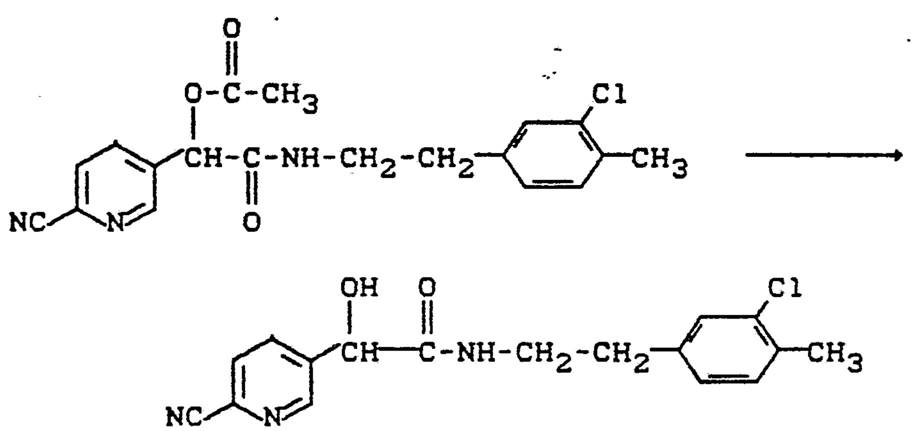

Die Verbindungen der Formel XI sind neu. Ihre Herstellung wird weiter unten beschrieben. Die Substituenten $R^1$ bis $R^{10}$, X und Y in den Verbindungen XI besitzen die bei den Verbindungen der Formel I angegebenen bevorzugten Bedeutungen. Im einzelnen seien folgende Verbindungen der Formel XI genannt:

(2-Amino-5-pyridyl)acetoxyessigsäure-(3-(4-methoxycarbonyl-methoxyphenyl)-2-propyl)amid
(2-Amino-3-chlor-5-pyridyl)acetoxyessigsäure-(3-(4-ethoxycarbonylphenyl)-2-propyl)amid
(2-Amino-3-cyano-5-pyridyl)acetoxyessigsäure-(3-(4-methoxycarbonylmethoxyphenyl)-2-propyl)amid
(3-Amino-2,4-dichlor-6-pyridyl)acetoxyessigsäure-(3-(4-ethoxycarbonylmethoxyphenyl)-2-propyl)amid
(2-(Cyano-3-amino-6-pyridyl)acetoxyessigsäure-(3-(4-methoxycarbonylphenyl)-2-propyl)amid
(3-Amino-4-cyano-6-pyridyl)acetoxyessigsäure-(3-(4-ethoxycarbonylmethoxyphenyl)-2-propyl)amid
(2,6-Dichlor-4-pyridyl)acetoxyessigsäure-(3-(4-methoxycarbonylphenyl)-2-propyl)amid
(2,6-Dichlor-4-pyridyl)acetoxyessigsäure-(3-(4-methoxycarbonylmethoxyphenyl)-2-propyl)amid.

Zur Abspaltung der Acetylgruppe werden anorganische Säuren verwendet. Hierzu zählen Halogenwasserstoffsäuren wie Salzsäure; Schwefelsäure, Phosphorsäure.

Das Verfahren wird durchgeführt, indem man die Verbindung XI in einem Verdünnungsmittel als Lösungsvermittler mit überschüssiger wäßriger Lösung der anorganischen Säure behandelt.

Die Reaktion wird bei Temperaturen von +20°C bis +150°C durchgeführt.

Es wird bevorzugt bei Normaldruck gearbeitet.

Als Verdünnungsmittel können alle inerten organischen Lösungsmittel, die mit Wasser mischbar sind, verwendet werden. Hierzu zählen Ether wie Tetrahydrofuran, Dioxan; Nitrile, wie Acetonitril; Amide wie Dimethylformamid; Alkohole wie Methanol, Ethanol; Dimethylsulfoxid.

Setzt man bei Verfahren 6) als Verbindung der Formel XII 2-Fluor-pyridin-5-aldehyd und als Isonitril der Formel XIII 3-(4-Methoxyphenyl)-2-propylisonitril ein, läßt sich das Verfahren durch das folgende Schema wiedergeben:

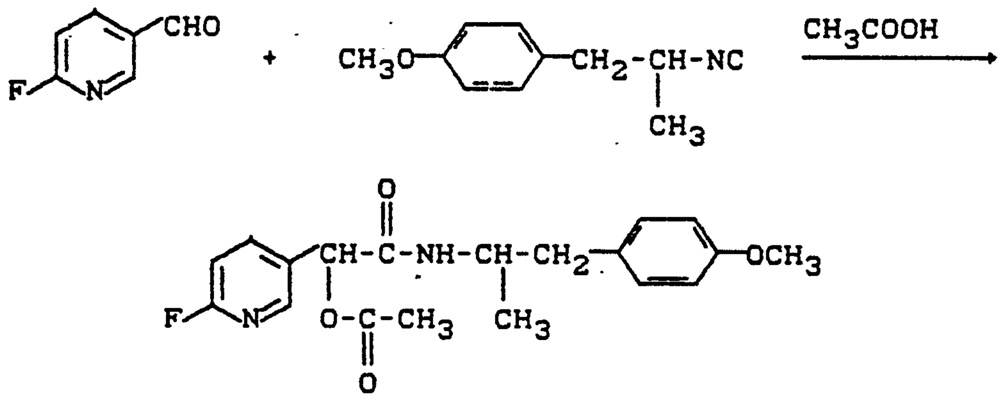


Die Herstellung der Aldehyde der Formel XII ist bereits bei Verfahren 2b) (oben) beschrieben. Die Substituenten $R^1$, $R^2$, $R^3$ in Formel XII besitzen die weiter oben bei den Verbindungen der Formel I angegebenen bevorzugten Bedeutungen. Im einzelnen seien die folgenden Verbindungen der Formel XII genannt:

2-Chlorpyridin-4-aldehyd
2-Cyanopyridin-4-aldehyd
2,6-Dichlorpyridin-4-aldehyd
2-Aminopyridin-5-aldehyd
2-Amino-3-chlorpyridin-5-aldehyd
2-Amino-3-cyanopyridin-5-aldehyd
2-Chlor-3-aminopyridin-6-aldehyd
2-Cyano-3-aminopyridin-6-aldehyd
2,4-Dichlor-3-aminopyridin-6-aldehyd
3-Amino-4-cyanopyridin-6-aldehyd.

Isonitrile der Formel XIII sind bekannt (I. Ugi et al., Angew. Chem. 77 (1965), 492) oder lassen sich analog zu bekannten Verbindungen herstellen. Die Substituenten $R^7$ bis $R^{10}$, X und Y besitzen die weiter oben bei den Verbindungen der Formel I angegebenen bevorzugten Bedeutungen. Im einzelnen seien folgende Verbindungen der Formel XIII genannt

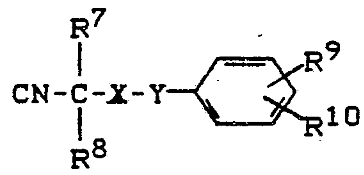

| $R^7$ | $R^8$ | X | Y | $R^9$ | $R^{10}$ |
|---|---|---|---|---|---|
| H | $CH_3$ | $-CH_2-$ | - | $4-COOCH_3$ | H |
| H | $CH_3$ | $-CH_2-$ | - | $4-OCH_2COOCH_3$ | H |
| $CH_3$ | H | $-CH_2-$ | O | $4-OCH_3$ | 3-Cl |
| $CH_3$ | H | $-(CH_2)_2-$ | O | $4-OCH_2CH_2OH$ | H |

Das Verfahren wird durchgeführt, indem man die Verbindung XII mit der doppelt molaren Menge des Isonitrils der Formel XIII und Essigsäure in einem Verdünnungsmittel umsetzt.

Die Reaktion wird bei Temperaturen von 0°C bis +150°C durchgeführt.

Es wird bevorzugt bei Normaldruck gearbeitet.

Als Verdünnungsmittel dienen alle inerten organischen Lösungsmittel. Hierzu gehören insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril und Benzonitril.

Die Wirkstoffe werden als Leistungsförderer bei Tieren zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier-und Hobbytieren verwendet.

Zu den Nutz-und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänze, Enten, Truthühner, Tauben, Fische wie z.B. Karpfen, Forellen, Lachse, Aale, Schleien, Hechte, Reptilien wie z.B. Schlangen und Krokodile.

Zu den Zier-und Hobbytieren zählen Säugetiere wie Hunde und Katzen, Vögel wie Papageien, Kanarienvögel, Fische wie Zier-und Aquarienfische z.B. Goldfische.

Die Wirkstoffe werden unabhängig vom Geschlecht der Tiere während allen Wachstums-und Leistungsphasen der Tiere eingesetzt. Bevorzugt werden die Wirkstoffe während der intensiven Wachstums-und Leistungsphase eingesetzt. Die intensive Wachstums-und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesunheitszustand der Tiere ab.

Die Verabreichung erfolgt oral oder parenteral in dafür geeigneten Formulierungen oder in reiner Form. Orale Formulierungen sind Pulver, Tabletten, Granulate, Doenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm - 100 %, bevorzugt von 0,01 ppm - 1 %.

Die Wirkstoffe können einmalig verabreicht werden. Die Wirkstoffe können aber auch während der ganzen oder während eines Teils der Wachstums-und Leistungsphase temporär oder kontinuierlich verabreicht werden.

Bei kontinuierlicher Verabreichung kann die Anwendung ein-oder mehrmals täglich in regelmäßigen oder unregelmäßigen Abständen erfolgen.

Die Wirkstoffe können in den Formulierungen allein oder in Mischung mit anderen leistungsfördernden Wirkstoffen, mineralischen Futtermitteln, Spurenelement-Verbindungen, Vitaminen, stickstoff liefernden Nicht-Protein-Verbindungen, Farbstoffen, Antioxidantien, Aromastoffen, Emulgatoren, Fließhilfsstoffen, Konservierungs-und Preßhilfsstoffen vorliegen.

Andere leistungsfördernde Wirkstoffe sind:
z.B. Antibiotika wie Tylosin und Virginiamycin.
Mineralische Futtermittel sind z.B. Dicalciumphosphat, Magnesiumoxid, Natriumchlorid.
Spurenelement-Verbindungen sind z.B. Eisenfumarat, Natriumjodid, Kobaltchlorid, Kupfersulfat, Zinkoxid.
Vitamine sind z.B. Vitamin A, Vitamin $D_3$, Vitamin E, B-Vitamine, Vitamin C.

Stickstoff-liefernde Nicht-Protein-Verbindungen sind z.B. Biuret, Harnstoff.
Farbstoffe sind z.B. Carotinoide wie Citranaxanthin, Zeaxanthin, Capsanthin.
Antioxidantien sind z.B. Ethoxyquin, Butylhydroxy-Toluol.
Aromastoffe sind z.B. Vanillin.
Emulgatoren sind z.B. Ester der Milchsäure, Lecithin.
Fließhilfsstoffe sind z.B. Natriumstearat, Calciumstearat.

Konservierungsstoffe sind z.B. Zitronensäure, Propionsäure.
Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Hernkunft wie Fleisch, Fette, Milch produkte, Knochenmehl, Fischprodukte, die Einzelfuttermittel wie Vitamine, Proteine, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie-und Proteinversorgung sowie der Versorgung mit Vitaminen, Mineralsalzen und Spurenelementen sicherstellt.

Die Kozentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das 10 ppm erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g des unten angegebenen Wirkstoff-Prämix ergeben nach sorgfältigem Mischen 1 kg Futter mit 10 ppm Wirkstoffgehalt.

In einem kg Vitamin-Mineralsind enthalten:
600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4 \times H_2O$, 140 mg Zn $SO_4 \times 7\ H_2O$, 100 mg Fe $SO_4 \times 7\ H_2O$ und 20 mg Cu $SO_4 \times 5\ H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das 8 ppm erfindungsgemäßen Wirkstoff enthält:

630 g Futtergetreideschrot (zusammengesetzt aus 200 g Mais, 150 g Gerste-, 150 g Hafer-und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter mit 8 ppm Wirkstoffgehalt.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammenetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt und die relative Gewichtszunahme im Vergleich zur unbehandelten Kontrolle errechnet.

Es werden die aus der Tabelle ersichtlichen Ergebnisse erhalten:

Tabelle 1

Ratten Fütterungsversuch

| Wirkstoff Beispiel Nr. | Wirkstoff Aufwand ppm | relative Gewichtszunahme % |
|---|---|---|
| 2 | 25 | 38 |
| 3 | 25 | 22 |
| 4 | 25 | 31 |
| 5 | 25 | 61 |
| 1 | 25 | 31 |
| 6 | 25 | 34 |

## Beispiele

Allgemeine Vorschrift für Verfahren 2a

Herstellung der Verbindungen der Formel I nach Verfahren 2a

10 mmol der Verbindung der Formel II werden bei 0°C portionsweise zu einer Lösung von 10 ml des Amins der Formel III in 15 ml absolutem Ethanol gegeben. Man läßt auf 10-15°C kommen und rührt bei dieser Temperatur eine Stunde nach. Dann wird wieder auf 0°C abgekühlt und portionsweise werden 600 mg (50 mmol) Natriumborhydrid zugegeben. Man rührt über Nacht bei Raumtemperatur nach. Nach Zugabe von 20 ml Wasser wird 30 min gerührt, eingedampft und zwischen Wasser und Essigester verteilt. Die organische Phase wird über Natriumsulfat getrocknet und eingedampft. Der Rückstand wird umkristallisiert.

Allgemeine Vorschrift für Verfahren 2b

Herstellung der Verbindungen der Formel I nach Verfahren 2b

0,1 mol der Verbindung der Formel IV und 0,11 mol des Amins der Formel III werden in 200 ml Methanol über Nacht unter Rückfluß erhitzt. Das Lösungsmittel und überschüssiges Amin werden abgezogen und der Rückstand umkristallisiert.

Allgemeine Vorschrift für Verfahren 2c

Herstellung der Verbindungen der Formel I nach Verfahren 2c

10 mmol der Verbindung der Formel V werden in 150 ml Ethanol gelöst und 20 ml des Amins der Formel III werden zugegeben, die Mischung wird 18 Stunden unter Rückfluß erhitzt. Dann werden Lösungsmittel und überschüssiges Amin abgezogen, und der Rückstand wird in 100 ml trockenem Ether aufgenommen. Das unlösliche Aminhydrohalogenid wird abfiltriert, die etherische Lösung mit Wasser gewaschen, über Natriumsulfat getrocknet und eingedampft. Das Rohprodukt wird umkristallisiert.

Allgemeine Vorschrift für Verfahren 2d

Herstellung der Verbindungen der Formel I nach Verfahren 2d

Zu 22 mmol der Verbindung VI in 10 ml absolutem Ethanol werden 22 mmol der Carbonylverbindung der Formel VII bei 0-5°C zugegeben. Man läßt auf Raumtemperatur kommen und rührt noch 30 Minuten nach. Die Lösung wird dann zu 0,15 g Adams-Katalysator (vorhydriert in 10 ml absolutem Ethanol) gegeben und die Mischung 4-5 Stunden bei 40°C und 50 atm Wasserstoffdruck hydriert. Nach Abfilterieren vom Katalysator wird eingedampft und umkristallisiert.

Allgemeine Vorschrift für Verfahren 2e

Herstellung der Verbindungen der Formel I nach Verfahren 2e

Zur Lösung von 10 mmol der Verbindung der Formel VIII in 50 ml Ethanol werden bei 10-15°C 15 mmol des Amins der Formel IX getropft. Man läßt auf Raumtemperatur kommen und rührt noch 15 min nach. Dann verdünnt man mit weiteren 100 ml Ethanol und gibt bei 0-5°C portionsweise 80 mmol Natriumborhydrid zu. Man läßt auf Raumtemperatur kommen und rührt über Nacht. Dann gibt man bei 10°C 200 ml Wasser zu, rührt 30 min., dampft das Ethanol ab und extrahiert den Rückstand dreimal mit je 50 ml Dichlormethan. Die vereinigten organischen Phasen werden mit 100 ml Wasser gewaschen, über Natriumsulfat getrocknet und eingedampft.

Allgemeine Vorschrift für Verfahren 2f

Herstellung der Verbindungen der Formel I nach Verfahren 2

Zu 12,4 ml einer 1 M Lösung von Boran in Tetrahydrofuran werden 2,3 mmol der Verbindung X in 30 ml absolutem Tetrahydrofuran getropft. Die Mischung wird eine 1 Stunde unter Rückfluß erhitzt, mit Eiswasser verdünnt und mit 50 ml 1N Salzsäure versetzt. Nach Abdampfen des organischen Lösungsmittels wird die saure wäßrige Lösung zweimal mit je 30 ml Ether extrahiert, dann mit gesättigter Natriumcarbonatlösung alkalisch gestellt und dreimal mit je 30 ml Essigester extrahiert. Die vereinigten Extrakte werden über Natriumsulfat getrocknet und eingedampft.

Analog zu den oben angegebenen Verfahren 2a - 2f werden die folgenden Verbindungen hergestellt:

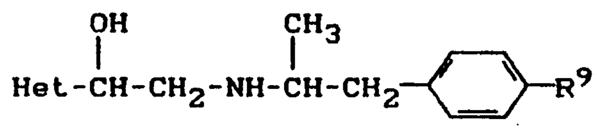

| Beispiel-Nr. | Het | $R^9$ | $^1H$-NMR ($CDCl_3$, δ[ppm]) |
|---|---|---|---|
| 1 | 2-Pyridyl | $COOCH_3$ | 1,05 (d, 3H); 2,6-3,15 (m, 5H); 3,9 (s, 3H); 4,75 (m, 1H); 7,1-7,4 (m, 4H); 7,7 (m, 1H); 7,9 (m, 2H); 8,5 (m, 1H). |
| 2 | 3-Pyridyl | $COOCH_3$ | 1,05 (dd, 3H); 2,1 (s(breit)1H); 2,5-3,05 (m, 6H); 3,9 (s, 3H); 4,6 (m, 1H); 7,95 (m, 2H); 8,5 (m, 2H). |
| 3 | 4-Pyridyl | $COOCH_3$ | 1,1 (dd, 3H); 2,25-3,1 (m, 6H); 3,95 (s, 3H); 4,6 (m,H); 7,2 (m, 3H); 8,0 (m, 3H); 8,5 (m, 2H). |
| 4 | 2-Pyridyl | $OCH_2COOCH_3$ | 1,05 (dd, 3H); 2,5-3,2 (m, 5H); 3,8 (s, 3H); 4,6 (s, 2H); 4,7 (m, 1H); 6,85 (m, 2H); 7,0 (m, 2H); 7,2 (m, 1H); 7,4 (m, 1H); 8,5 (m, 1H). |

| Beispiel-Nr. | Het | $R^9$ | $^1H$-NMR ($CDCl_3$, δ[ppm]) |
|---|---|---|---|
| 5 | 3-Pyridyl | $OCH_2COOCH_3$ | 1,1 (d, 3H); 2,5-3,2 (m, 6H); 3,8 (s, 3H); 4,6 (s, 2H), 4,7 (m, 1H); 6,85 (m, 2H); 7,0-7,2 (m, 3H); 7,7 (m, 1H); 8,5 (m, 2H). |
| 6 | 4-Pyridyl | $OCH_2COOCH_3$ | 1,05 (d, 3H); 2,5-3,05 (m, 5H); 3,8 (s, 3H); 4,6 (m, 1H); 4,65 (s, 2H); 6,8 (m, 2H); 7,0-7,3 (m, 4H); 8,5 (m, 2H). |
| 7 | 2,6-Dichlor-4-pyridyl | $OCH_2CH_2OH$ | 1,1(dd,3H); 2,2 (s (breit), 1H); 2,5-3,1 (m, 5H); 4,0 (dd, 2H); 4,1 (dd, 2H); 4,6 (m, 1H); 6,8-7,2 (m, 6H). |
| 8 | 2-Amino-3-chlor-5-pyridyl | $OCH_2CH_2OH$ | 1,1 (d, 3H); 2,2 (s(breit), 1H); 2,5-3,0 (m, 5H); 4,0 (dd, 2H); 4,5 (m, 1H); 5,0 (s (breit), 2H); 6,9 (m, 2H; 7,1 (m, 2H); 7,5 (m, 1H); 7,8 (m, 1H). |
| 9 | 2-Amino-3-chlor-5-pyridyl | $OCH_2COOCH_3$ | 1,1 (dd, 3H); 2,1 (s (breit), 1H); 2,5-3,0 (m, 5H); 3,8 (s, 3H); 4,5 (m, 1H); 4,6 (s, 2H); 4,9 (s(breit), 2H); 6,9 (m, 2H); 7,1 (m, 2H); 7,5 (m, 1H); 7,9 (m, 1H). |
| 10 | 2-Chlor-4-pyridyl | $OCH_2CH_2OH$ | 1,1 (d, 3H); 2,2 (s (breit), 1H); 2,5-3,0 (m, 5H); 3,9 (dd, 2H); 4,1 (dd, 2H); 4,6 (m, 1H); 6,9-7,2 (m, 6H); 8,3 (m, 1H). |

Allgemeine Vorschrift für Verfahren 4

Herstellung der Verbindungen der Formel X nach Verfahren 4

Eine Suspension von 10 mmol der Verbindung XI in einer Mischung aus 37 ml Methanol, 37 ml Wasser und 18 ml 2,5 N Salzsäure wird eine Stunde zum Sieden erhitzt. Dann wird das Methanol abgezogen und der ausgefallene Feststoff abgesaugt.

Allgemeine Vorschrift für Verfahren 6

Herstellung von Verbindungen der Formel XI nach Verfahren 6

Eine Mischung von 10 mmol der Verbindung XII, 20 mmol der Verbindung XIII und 20 mmol Eisessig in 50 ml trockenem Chloroform wird zwei Stunden unter Rückfluß erhitzt. dann wird mit 5 %iger wäßriger $NaHCO_3$-Lösung gewaschen, über $Na_2SO_4$ getrocknet und eingedampft.

Beispiel für die Herstellung von Verbindungen der Formel VI:

1-(2,6-Dichlor-4-pyridyl)-2-aminoethanol

Zur Lösung von 11,4 g (56 mmol) (2,6-Dichlor-4-pyridyl)cyanhydrin in 200 ml abs. Tetrahydrofuran werden bei Raumtemperatur 230 ml (230 mmol) einer 1 M Lösung von Boran in Tetrahydrofuran getropft. Nach beendeter Zugabe wird eine Stunde unter Rückfluß erhitzt, dann über Nacht bei Raumtemperatur nachgerührt. Nach Ansäuern mit konz. Salzsäure auf pH 1 wird 30 min. gerührt und eingedampft. Der Rückstand wird in wenig Wasser aufgenommen, filtriert, mit verdünnter Natronlauge auf pH 3 gestellt und mit Essigester gewaschen (3 × 50 ml). Dann wird alkalisch gestellt und mit Essigester (3 × 100 ml) das Produkt extrahiert. Nach Trocknen und Eindampfen erhält man 9,1 g (73 %), Fp. 120°C (Z.).

Beispiel für die Herstellung der Verbindungen der Formel XIV:

(2,6-Dichlor-4-pyridyl)cyanhydrin

Zur Lösung von 5 g (28,4 mmol) 2,6-Dichlorpyridin-4-aldehyd in 30 ml Ether werden 10 ml 40 %ige wässrige Natriumhydrogensulfit-Lösung gegeben. Dann werden auf einmal 3,85 g Natriumcyanid in Form einer gesättigten wäss rigen Lösung zugegeben. Nach einer Stunde Rühren wird die etherische Phase abgetrennt und die wässrige noch einmal mit 20 ml Ether extrahiert. Die vereinigten organischen Phasen werden getrocknet und eingedampft.

Ausbeute: 5,7 g (quant.), Fp 140°C (Z.).

**Ansprüche**

1. Neue Verbindungen der Formel I

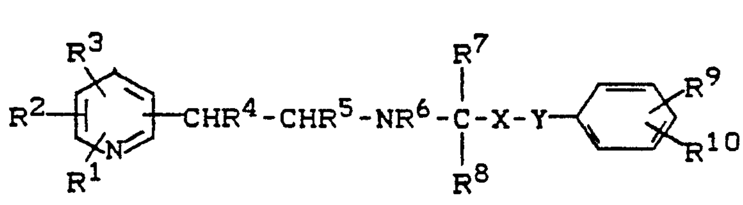

(I)

in welcher

$R^1$ für Wasserstoff, Alkyl, Halogen, Halogenalkyl, Hydroxy, Cyano, Alkoxycarbonyl, Aminocarbonyl, Mono- und Dialkylaminocarbonyl, Alkoxy Halogenalkoxy, Halogenalkylthio, $NHSO_2$-Alkyl steht,

$R^2$ für Wasserstoff, Hydroxy, Alkoxy oder den Rest $-NR^{11}R^{12}$ steht,

$R^3$ für die bei $R^1$ angegebenen Reste steht,

$R^4$ für Hydroxy, Acyloxy oder Alkoxy steht,

$R^5$ für Wasserstoff oder Alkyl steht,

$R^6$ für Wasserstoff oder Alkyl steht,

$R^7$ für Wasserstoff oder Alkyl steht,

$R^8$ für Wasserstoff oder Alkyl steht,

X für $C_1$-$C_{10}$-Alkylen oder eine direkte Bindung steht,

Y für Sauerstoff oder eine direkte Bindung steht,

$R^9$ für Wasserstoff, $C_1$-$C_{10}$-Alkyl, welches gegebenenfalls durch Hydroxy, Alkoxy, Acyloxy oder den Rest $-NR^{13}R^{14}$ substituiert ist, sowie für den Rest $COR^{15}$ oder den Rest $O$-$Z$-$R^{16}$ steht,

Z für $C_1$-$C_{10}$-Alkylen, -Alkenylen oder -Alkinylen steht,

$R^{10}$ für Wasserstoff, Halogen, Alkyl, Alkoxy, Hydroxy oder den Rest $COR^{15}$ steht,

$R^{11}$ für Wasserstoff oder Alkyl steht,

$R^{12}$ für Wasserstoff, Alkyl, Halogenalkyl oder Acyl steht,

$R^{13}$ für Wasserstoff, gegebenenfalls substituiertes Alkyl, gegebenenfalls substituiertes Aryl steht,

$R^{14}$ für die bei $R^{13}$ angegebenen Reste steht,

$R^{15}$ für Hydroxy, Alkoxy oder den Rest $NR^{13}R^{14}$ steht,

$R^{16}$ für Hydroxy, Alkoxy, Acyloxy, den Rest $NR^{13}R^{14}$ oder den Rest $COR^{15}$ steht,

sowie ihre physiologische verträglichen Salze und N-Oxide.

2. Verfahren zur Herstellung der Verbindungen der Formel I,

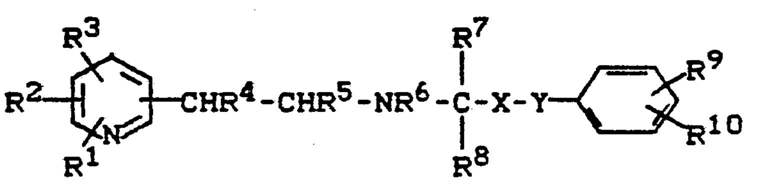

(I)

in welcher

$R^1$ für Wasserstoff, Alkyl, Halogen, Halogenalkyl, Hydroxy, Cyano, Alkoxycarbonyl, Aminocarbonyl, Mono- und Dialkylaminocarbonyl, Alkoxy, Halogenalkoxy, Halogenalkylthio, $NHSO_2$-Alkyl steht,

$R^2$ für Wasserstoff, Hydroxy, Alkoxy oder den Rest $-NR^{11}R^{12}$ steht,

$R^3$ für die bei $R^1$ angegebenen Reste steht,

$R^4$ für Hydroxy, Acyloxy oder Alkoxy steht,

$R^5$ für Wasserstoff oder Alkyl steht,

$R^6$ für Wasserstoff oder Alkyl steht,

$R^7$ für Wasserstoff oder Alkyl steht,

$R^8$ für Wasserstoff oder Alkyl steht,

X für $C_1$-$C_{10}$-Alkylen oder eine direkte Bindung steht,

Y für Sauerstoff oder eine direkte Bindung steht,

$R^9$ für Wasserstoff, $C_1$-$C_{10}$-Alkyl, welches gegebenenfalls durch Hydroxy, Alkoxy, Acyloxy oder den Rest $-NR^{13}R^{14}$ substituiert ist, sowie für den Rest $COR^{15}$ oder O-Z-$R^{16}$ steht,

Z für $C_1$-$C_{10}$-Alkylen, -Alkenylen oder -Alkinylen steht,

$R^{10}$ für Wasserstoff, Halogen, Alkyl, Alkoxy, Hydroxy oder den Rest $COR^{15}$ steht,

$R^{11}$ für Wasserstoff oder Alkyl steht,

$R^{12}$ für Wasserstoff, Alkyl, Halogenalkyl oder Acyl steht,

$R^{13}$ für Wasserstoff, gegebenenfalls substituiertes Alkyl, gegebenenfalls substituiertes Aryl steht,

$R^{14}$ für die bei $R^{13}$ angegebenen Reste steht,

$R^{15}$ für Hydroxy, Alkoxy oder den Rest $NR^{13}R^{14}$ steht,

$R^{16}$ für Hydroxy, Alkoxy, Acyloxy, den Rest $NR^{13}R^{14}$ oder den Rest $COR^{15}$ steht,

dadurch gekennzeichnet, daß man

a) Halogenmethylketone der Formel II,

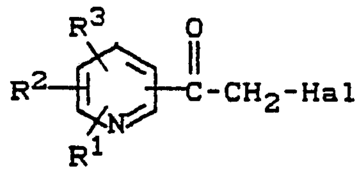

(II)

in welcher

$R^1$, $R^2$, $R^3$ die oben angegebene Bedeutung haben und Hal für Halogen steht,

mit Aminen der Formel III

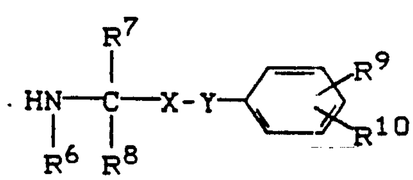

(III)

in welcher

$R^6$ bis $R^{10}$, X und Y die oben angegebene Bedeutung haben,

umsetzt und anschließend die Carbonylgruppe reduziert, oder
b) Epoxide der Formel IV

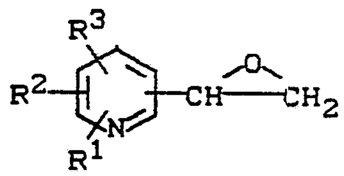

(IV)

in welcher

$R^1$, $R^2$, $R^3$ die oben angegebene Bedeutung haben,

mit Aminen der Formel III

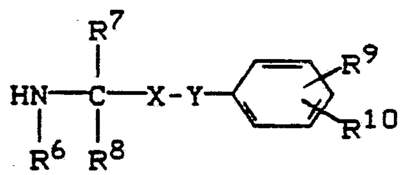

(III)

in welcher

$R^6$ bis $R^{10}$, X und Y die oben angegebene Bedeutung haben,

umsetzt, oder
c) $\beta$-Halogenethylverbindungen der Formel V

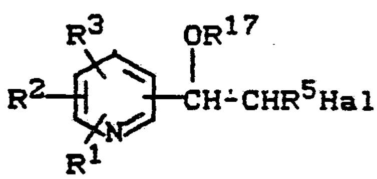

(V)

in welcher

$R^1$, $R^2$, $R^3$ und $R^5$ die oben angegebene Bedeutung haben,

Hal für Halogen steht,

$R^{17}$ für Wasserstoff oder $C_1$-$C_4$-Alkyl steht,

mit Aminen der Formel III

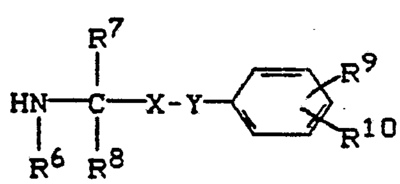

(III)

in welcher

$R^6$ bis $R^{10}$, X und Y die oben angegebene Bedeutung haben,

umsetzt, oder

d) für den Fall, daß in Formel I $R^6$ und $R^7$ für Wasserstoff stehen,

Verbindungen der Formel VI

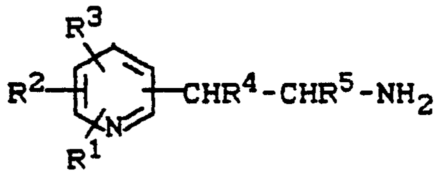

(VI)

in welcher

$R^1$ bis $R^5$ die oben angegebene Bedeutung haben,

mit Ketonen der Formel VII

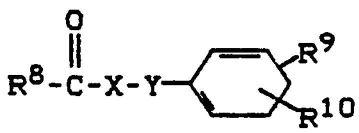

(VII)

in welcher

$R^8$, $R^9$, $R^{10}$, X und Y die oben angegebene Bedeutung haben,

unter reduzierenden Bedingungen umsetzt, oder

e) für den Fall daß in Formel I $R^4$ für Hydroxy steht und $R^5$ und $R^6$ für Wasserstoff stehen,

Verbindung der Formel VIII

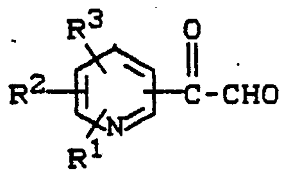

(VIII)

in welcher

$R^1$, $R^2$, $R^3$ die oben angegebene Bedeutung haben,

mit Aminen der Formel IX

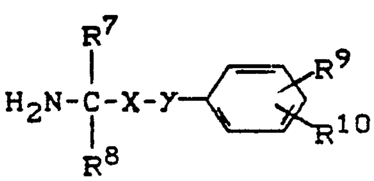

(IX)

in welcher

$R^7$ bis $R^{10}$ die oben angegebene Bedeutung haben,

unter reduzierenden Bedingungen umsetzt, oder

f) für den Fall, daß in Formel I $R^4$ für Hydroxy und $R^6$ für Wasserstoff stehen,

Verbindungen der Formel X

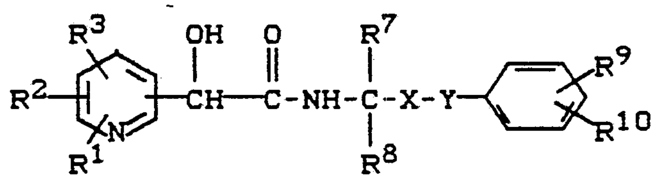

(X)

in welcher

$R^1$, $R^2$, $R^3$, $R^7$, $R^8$, $R^9$, $R^{10}$, X und Y die oben angegebene Bedeutung haben,

reduziert.

3. Neue Verbindung der Formel X

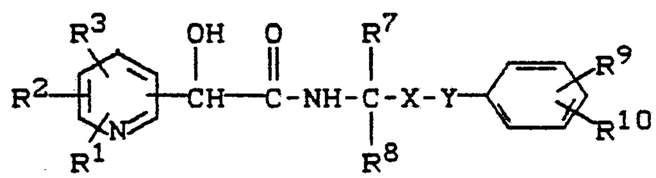

(X)

in welcher

$R^1$ für Wasserstoff, Alkyl, Halogen, Halogenalkyl, Hydroxy, Cyano, Alkoxycarbonyl, Aminocarbonyl, Mono- und Dialkylaminocarbonyl, Alkoxy Halogenalkoxy, Halogenalkylthio, $NHSO_2$-Alkyl steht,

$R^2$ für Wasserstoff, Hydroxy, Alkoxy oder den Rest $-NR^{11}R^{12}$ steht,

$R^3$ für die bei $R^1$ angegebenen Reste steht,

$R^7$ für Wasserstoff oder Alkyl steht,

$R^8$ für Wasserstoff oder Alkyl steht,

X für $C_1$-$C_{10}$-Alkylen oder eine direkte Bindung steht,

Y für Sauerstoff oder eine direkte Bindung steht,

$R^9$ für Wasserstoff, $C_1$-$C_{10}$-Alkyl, welches gegebenenfalls durch Hydroxy, Alkoxy, Acyloxy oder den Rest $-NR^{13}R^{14}$ substituiert ist, sowie für den Rest $COR^{15}$ oder den Rest O-Z-$R^{16}$ steht,

Z für $C_1$-$C_{10}$-Alkylen, -Alkenylen oder -Alkinylen steht,

$R^{10}$ für Wasserstoff, Halogen, Alkyl, Alkoxy, Hydroxy oder den Rest $COR^{15}$ steht,

$R^{11}$ für Wasserstoff oder Alkyl steht,

$R^{12}$ für Wasserstoff, Alkyl, Halogenalkyl oder Acyl steht,

$R^{13}$ für Wasserstoff, gegebenenfalls substituiertes Alkyl, gegebenenfalls substituiertes Aryl steht,

$R^{14}$ für die bei $R^{13}$ angegebenen Reste steht,

$R^{15}$ für Hydroxy, Alkoxy oder den Rest $NR^{13}R^{14}$ steht,

$R^{16}$ für Hydroxy, Alkoxy, Acyloxy, den Rest $NR^{13}R^{14}$ oder den Rest $COR^{15}$ steht.

4. Verfahren zur Herstellung der Verbindungen der Formel X, gemäß Anspruch 3, dadurch gekennzeichnet, daß man Verbindungen der Formel XI

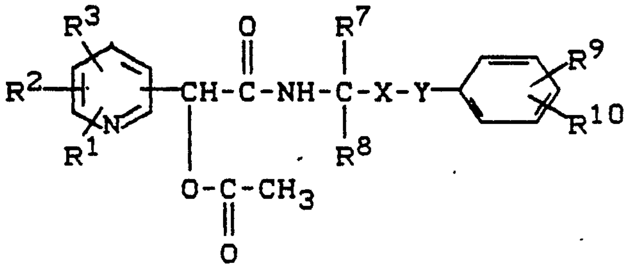

(XI)

in welcher

$R^1$ bis $R^{10}$, X und Y die in Anspruch 3 angegebene Bedeutung haben,

hydrolysiert.

5. Neue Verbindung der Formel XI

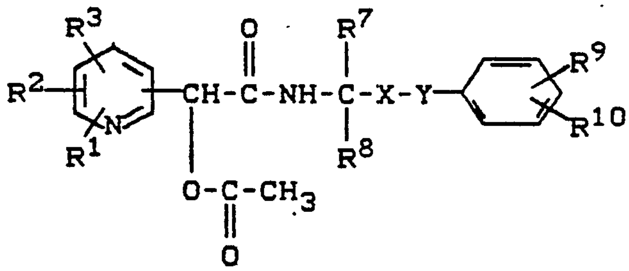

(XI)

in welcher

$R^1$ für Wasserstoff, Alkyl, Halogen, Halogenalkyl, Hydroxy, Cyano, Alkoxycarbonyl, Aminocarbonyl, Mono- und Dialkylaminocarbonyl, Alkoxy Halogenalkoxy, Halogenalkylthio, $NHSO_2$-Alkyl steht,

$R^2$ für Wasserstoff, Hydroxy, Alkoxy oder den Rest $-NR^{11}R^{12}$ steht,

$R^3$ für die bei $R^1$ angegebenen Reste steht,

$R^7$ für Wasserstoff oder Alkyl steht,

$R^8$ für Wasserstoff oder Alkyl steht,

X für ~~$C_1$-$C_{10}$~~-Alkylen oder eine direkte Bindung steht,

Y für Sauerstoff oder eine direkte Bindung steht,

$R^9$ für Wasserstoff, $C_1$-$C_{10}$-Alkyl, welches gegebenenfalls durch Hydroxy, Alkoxy, Acyloxy oder den Rest $-NR^{13}R^{14}$ substituiert ist, sowie für den Rest $COR^{15}$ oder den Rest O-Z-$R^{16}$ steht,

Z für $C_1$-$C_{10}$-Alkylen, -Alkenylen oder -Alkinylen steht,

$R^{10}$ für Wasserstoff, Halogen, Alkyl, Alkoxy, Hydroxy oder den Rest $COR^{15}$ steht,

$R^{11}$ für Wasserstoff oder Alkyl steht,

$R^{12}$ für Wasserstoff, Alkyl, Halogenalkyl oder Acyl steht,

$R^{13}$ für Wasserstoff, gegebenenfalls substituiertes Alkyl, gegebenenfalls substituiertes Aryl steht,

$R^{14}$ für die bei $R^{13}$ angegebenen Reste steht,

$R^{15}$ für Hydroxy, Alkoxy oder den Rest $NR^{13}R^{14}$ steht,

$R^{16}$ für Hydroxy, Alkoxy, Acyloxy, den Rest $NR^{13}R^{14}$ oder den Rest $COR^{15}$ steht.

6. Verfahren zur Herstellung der Verbindungen der Formel XI, gemäß Anspruch 5 dadurch gekennzeichnet, daß man Aldehyde der Formel XII

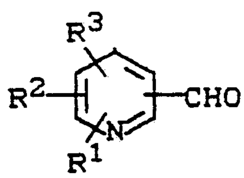

(XII)

in welcher

$R^1$, $R^2$, $R^3$ die oben angegebene Bedeutung haben,

mit Isonitrilen der Formel XIII

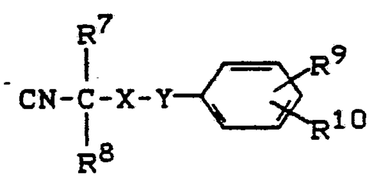

(XIII)

in welcher

$R^7$ bis $R^{10}$, X und Y die oben angegebene Bedeutung haben,

in Gegenwart von Essigsäure umsetzt.

7. Mittel zur Leistungsförderung von Tieren, gekennzeichnet durch einen Gehalt an Heteroarylethylaminen der Formel I gemäß Anspruch 1.

8. Tierfutter, Trinkwasser für Tiere, Zusätze für Tierfutter und Trinkwasser für Tiere, gekennzeichnet durch einen Gehalt an Heteroarylethylaminen der Formel I gemäß Anspruch 1.

9. Verwendung von Heteroarylethylaminen der Formel I gemäß Anspruch 1 zur Leistungsförderung von Tieren.

10. Verfahren zur Herstellung von Mitteln zur Leistungsförderung von Tieren, dadurch gekennzeichnet, daß man Heteroarylethylaminen der Formel I gemäß Anspruch 1 mit Streck-und/oder Verdünnungsmitteln versetzt.

11. Verfahren zur Herstellung von Tierfutter, Trinkwasser für Tiere oder Zusätze für Tierfutter und Trinkwasser für Tiere, dadurch gekennzeichnet, daß man Heteroarylethylamine der Formel I gemäß Anspruch 1 mit Futtermitteln oder Trinkwasser und gegebenenfalls weiteren Hilfsstoffen vermischt.